# EUROPEAN PATENT SPECIFICATION

(11) **EP 2 224 972 B1**
(45) Date of publication and mention of the grant of the patent: **24.01.2018**
(21) Application number: 08861568.7
(22) Date of filing: 17.12.2008
(51) Int. Cl.: A61L 29/08

(54) **SPIRAL CUT HYPOTUBE**
SPIRALFÖRMIG GESCHNITTENES HYPOROHR
HYPOTUBE À DÉCOUPE EN SPIRALE

(30) Priority: 18.12.2007 US 959163
(43) Date of publication of application: 08.09.2010
(73) Proprietor: Boston Scientific Scimed, Inc., Maple Grove, MN 55311 (US)
(72) Inventor: SUTERMEISTER, Derek, Plymouth, Minnesota (US); RASSAT, Jay, Buffalo, Minnesota 55313 (US); EIDENSCHINK, Tracee E. J., Wayzata, Minnesota 55391 (US); BELTON, Craig, Eden Prairie, Minnesota (US)
(74) Representative: Vossius & Partner Patentanwälte Rechtsanwälte mbB
(86) International application number: PCT/US2008/087216
(87) International publication number: WO 2009/079574

(56) References cited:
- WO-A1-2007/057132
- US-A1- 2005 165 439
- US-A1- 2007 247 033
- US-A1- 2007 250 036

## Description

### Technical Field

The present invention relates generally to medical devices and more particularly to medical devices that may include or be formed from a spiral cut hypotube.

### Background

Medical devices such as catheters may be subject to a number of often conflicting performance requirements such as flexibility, strength, minimized exterior diameter, maximized interior diameter, and the like. In particular, often times there is a balance between a need for flexibility and a need for strength. Therefore, a need remains for improved medical devices such as catheters that are configured for an optimal balance between flexibility, strength, and other desired properties.

### Summary

The present invention pertains to improved medical devices providing advantages in flexibility, strength and other desired properties as described in claim 1. Accordingly, described herein is a medical device such as a catheter that has an elongate shaft that includes a hypotube having a helical cutting formed within the hypotube. The elongate shaft may define a lumen that extends within the elongate shaft. An electroactive polymer may be disposed over at least a portion of the hypotube. Described herein is a medical device that includes a spiral cut hypotube having a constant cutting, or relaxed, pitch. The medical device may be configured to reversibly and temporarily alter the pitch of at least a portion of the spiral cut hypotube. Described herein is a medical device that includes a spiral cut hypotube having a constant pitch. The medical device may be configured to reversibly and/or temporarily alter a compressive strength of at least a portion of the spiral cut hypotube.

The above summary of the present invention is not intended to describe each disclosed embodiment or every implementation of the present invention. The Figures, Detailed Description and Examples which follow more particularly exemplify these embodiments.

### Brief Description of the Figures

The invention may be more completely understood in consideration of the following detailed description of various embodiments of the invention in connection with the accompanying drawings, in which:
Figure 1 is a side elevation view of a catheter in accordance with an embodiment of the invention;
Figure 2 is a side elevation view of a spiral cut hypotube in accordance with an embodiment of the invention;
Figure 3 is a side elevation view of a spiral cut hypotube in accordance with an embodiment of the invention;
Figure 4 is a side elevation view of a spiral cut hypotube in accordance with an embodiment of the invention;
Figure 5 is a side elevation view of a spiral cut hypotube in accordance with an embodiment of the invention;
Figure 6 is a side elevation view of a spiral cut hypotube in accordance with an embodiment of the invention;
Figure 7 is a side elevation view of a spiral cut hypotube in accordance with an embodiment of the invention;
Figure 8 is a side elevation view of a spiral cut hypotube in accordance with an embodiment of the invention; and
Figure 9 is a side elevation view of a spiral cut hypotube in accordance with an embodiment of the invention.

### Detailed Description

For the following defined terms, these definitions shall be applied, unless a different definition is given in the claims or elsewhere in this specification.

All numeric values are herein assumed to be modified by the term "about", whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure.

The recitation of numerical ranges by endpoints includes all numbers within that range (e.g., 1 to 5 includes 1, 1.5, 2, 2.75, 3, 3.80, 4, and 5).

As used in this specification and the appended claims, the singular forms "a", "an", and "the" include plural referents unless the content clearly dictates otherwise. As used in this specification and the appended claims, the term "or" is generally employed in its sense including "and/or" unless the content clearly dictates otherwise.

The following description should be read with reference to the drawings wherein like reference numerals indicate like elements throughout the several views. The drawings, which are not necessarily to scale, depict illustrative embodiments of the claimed invention.

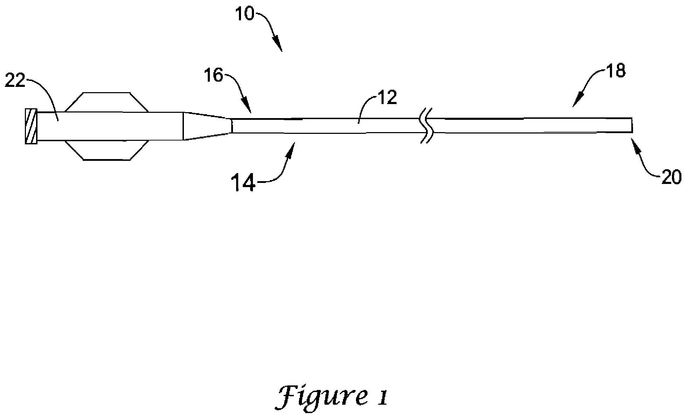

Figure 1 is a plan view of a catheter 10 in accordance with an embodiment of the present invention. The catheter 10 can be any of a variety of different catheters. In some embodiments, the catheter 10 can be an intravascular catheter. Examples of intravascular catheters include balloon catheters, atherectomy catheters, drug delivery catheters, stent delivery catheters, diagnostic catheters and guide catheters. The intravascular catheter 10 can be sized in accordance with its intended use. The catheter 10 can have a length that is in the range of about 100 to 150 centimeters and can have any useful diameter. Except as described herein, the intravascular catheter 10 can be manufactured using conventional techniques.

In the illustrated embodiment, the intravascular catheter 10 includes an elongate shaft 12 that has a proximal region 14 defining a proximal end 16 and a distal region 18 defining a distal end 20. A hub and strain relief assembly 22 can be connected to the proximal end 16 of the elongate shaft 12. The hub and strain relief assembly 22 may largely be of conventional design and can be attached using conventional techniques, apart from being adapted to accommodate electrical contacts that are in electrical communication with the electrodes that will be discussed in greater detail with respect to subsequent Figures. In some instances, it is contemplated that hubs such as those used in electrophysiology catheters may be useful.

The elongate shaft 12 can include one or more shaft segments having varying degrees of flexibility. For example, the elongate shaft may include a relatively stiff proximal portion, a relatively flexible distal portion and an intermediate position disposed between the proximal and distal portions having a flexibility that is intermediate to both.

In some cases, the elongate shaft 12 may be formed of a single polymeric layer. In some instances, the elongate shaft 12 may include an inner liner such as an inner lubricious layer and an outer layer. In some cases, the elongate shaft 12 may include a reinforcing braid layer disposed between the inner and outer layers. The elongate shaft 12 is considered herein as generically representing a catheter to which various elements can be added to provide the catheter 10 with desirable parameters such as flexibility and/or pushability.

If the elongate shaft 12 includes an inner liner, the inner liner can include or be formed from a coating of a material having a suitably low coefficient of friction. Examples of suitable materials include perfluoro polymers such as polytetrafluoroethylene (PTFE), better known as TEFLON®, high density polyethylene (HDPE), polyarylene oxides, polyvinylpyrolidones, polyvinylalcohols, hydroxy alkyl cellulosics, algins, saccharides, caprolactones, and the like, and mixtures and combinations thereof.

The elongate shaft 12 can include, as an outer layer or layers, any suitable polymer that will provide the desired strength, flexibility or other desired characteristics. Polymers with low durometer or hardness can provide increased flexibility, while polymers with high durometer or hardness can provide increased stiffness. In some embodiments, the polymer material used is a thermoplastic polymer material. Some examples of suitable materials include polyurethane, elastomeric polyamides, block polyamide/ethers (such as PEBAX®), silicones, and co-polymers. The outer polymer layer 32 can be a single polymer, multiple longitudinal sections or layers, or a blend of polymers. By employing careful selection of materials and processing techniques, thermoplastic, solvent soluble, and thermosetting variants of these materials can be employed to achieve the desired results. In some instances, a thermoplastic polymer such as a co-polyester thermoplastic elastomer, for example, available commercially under the ARNITEL® name, can be used.

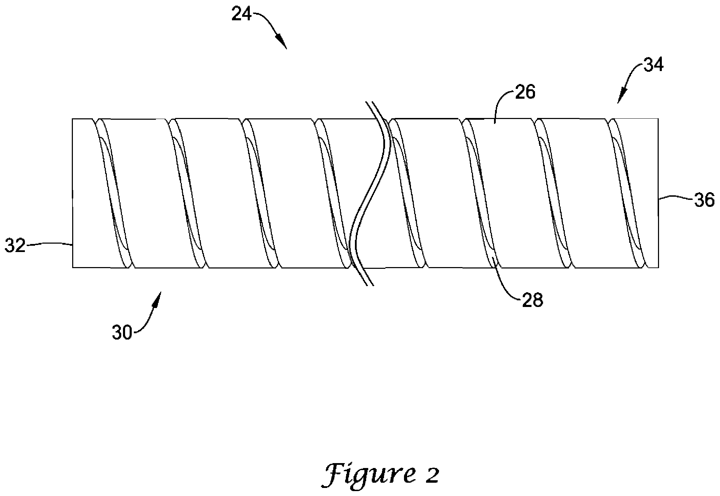

Figures 2 through 9 illustrate various examples of spiral cut hypotubes in accordance with illustrative but non-limiting examples of the present invention. It is considered that the catheter 10 may include or be formed from any of these hypotubes. It should be noted that while these hypotubes are described with respect to catheters, they are equally applicable to other medical devices such as guidewires.

Figure 2 illustrates a spiral cut hypotube 24 that includes a hypotube body 26 and a helical cutting 28 formed within the hypotube body 26. The spiral cut hypotube 24 has a proximal region 30 defining a proximal end 32 and a distal region 34 defining a distal end 36. The hypotube body 26 may be formed of any suitable polymeric or metallic material. In some cases, the hypotube body 26 may be formed of a suitably stiff polymer such as carbon fibers, liquid crystal polymers, polyimide, and the like. In some instances, the hypotube body 26 may be formed of a metallic material such as stainless steel or a nickel-titanium alloy such as Nitinol or other metallic or polymeric shape-memory material. The hypotube body 26 may include a combination of metal tubes and polymer tubes, if desired.

The hypotube body 26 may be formed having any desired length, width and material thickness as required to satisfy the requirements of any particular application. The helical cutting 28 may be formed using any suitable technique, such as saw cutting, a laser, or even by electrical discharge machining (EDM). Additional suitable techniques include chemical etching and abrasive grinding. In some instances, the helical cutting 28 may be formed such that the spiral cut hypotube 24 has a uniform pitch, or distance between windings, an entire length of the spiral cut hypotube 24 or at least a substantially portion thereof. In some cases, the helical cutting 28 may have a constant or at least a substantially constant slot width over an entire length of the spiral cut hypotube 24 or at least over a substantial portion thereof.

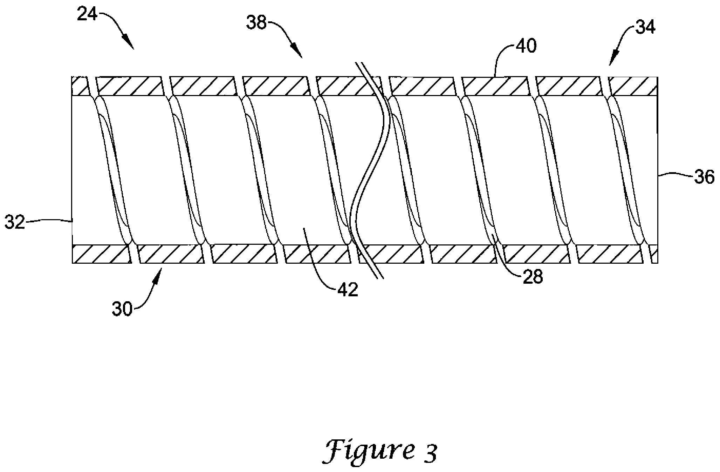

In some cases, it may be advantageous to provide structure and/or techniques that may reversibly and/or temporarily change certain properties of the spiral cut hypotube 24. Examples of properties that may, in some cases, be changed or altered include flexibility and pushability. In a spiral cut structure, winding and/or unwinding of the individual turnings may impact flexibility and/or pushability. Figures 3 through 9 provide structure and/or techniques to alter certain properties of the spiral cut hypotube 24.

Figure 3 shows an assembly 38 in which an electroactive polymer 40 has been added to the spiral cut hypotube 24. In Figure 3, the electroactive polymer 40 has been provided along at least some of the individual turnings 42 of the hypotube body 26 such that the electroactive polymer 40 does not or at least does not substantially cover or overlap the helical cutting 28. While the electroactive polymer 40 is shown as covering substantially all of the individual turnings 42 of the hypotube body 26, it will be recognized that the electroactive polymer 40 may instead be disposed only along a portion of the length of the hypotube body 26, or perhaps within two or more distinct segments or portions along the length of the hypotube body 26.

In some cases, the electroactive polymer 40 may include or be a doped polymer that undergoes volume or configuration changes upon oxidation and reduction, such as may occur when the polymer is subjected to an electrical field driving the ions into or out of the polymer. Oxidation and reduction may cause ions to be either inserted into the polymer, thereby increasing the volume of the polymer, or to be removed from the polymer, thereby decreasing its volume.

In some instances, the electroactive polymer 40 may be a polymer that can, when subjected to a potential difference, accommodate ions which may cause the electroactive polymer to swell. By reversing the potential difference, the ions that previously entered the polymer will exit the polymer and the polymer may return to its previous size, volume or configuration. In some cases, the electroactive polymer 40 may be held at an intermediate size, volume or configuration.

In particular, halting the potential difference being applied to the electroactive polymer 40 will permit ions already within the polymer to remain there, but additional ions will not enter. Reversing the potential difference will cause the previously entered ions to exit the polymer. It should be recognized, therefore, that the relative amount of ions entering or exiting the electroactive polymer 40 may be controlled by controlling the potential difference applied to the electroactive polymer.

In some instances, the electroactive polymer 40 may be doped with a large, immobile anion A- and may be positioned in contact with an electrolyte that includes a small mobile cation M+, in which case cations are inserted and de-inserted. The electroactive polymer 40, in this case, expands in volume in its reduced state (a negative potential). This can be represented as the following redox (oxidation-reduction) reaction:

P⁺(A⁻) + M⁺(aq) + e⁻ ⇔ P⁰A⁻M⁺).

In some instances, the electroactive polymer 40 can be polypyrrole that has been doped with dodecyl benzene sulfonate (DBS), and can be placed in contact with an aqueous electrolyte of 0.1 molar NaDBS (sodium dodecyl benzene sulfonate). In this case, DBS is the large, immobile anion and Na⁺, possibly hydrated, is the small cation that is inserted and or de-inserted into the polymer. During reduction, sodium cations move into the polypyrrole to achieve charge neutrality within the polypyrrole. On oxidation, conversely, the sodium cations are expelled from the polypyrrole.

Polypyrrole and NaDBS have the following chemical structures, respectively:

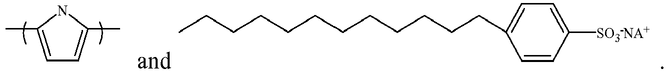

As noted, sodium cations can be provided by contacting the polypyrrole with an NaDBS electrolyte solution. However, in some instances, any variety of different aqueous salt solutions are useful. In particular, bodily fluids such as blood plasma and urine are effective.

Thus, in some instances, the electroactive polymer 40 may be adapted to accommodate ions from an electrolyte solution provided within the spiral cut hypotube 24. In some cases, the electroactive polymer 40 may be adapted to accommodate ions from a patient's own blood. Ions in general, and particularly cations, may flow (as a result of an appropriate potential difference) from either an electrolyte solution such as NaDBS or from a patient's blood into the electroactive polymer 40, thereby swelling or otherwise activating the electroactive polymer.

As noted, it is useful to provide a voltage or potential difference in order to drive the redox reaction discussed above. The oxidized state, in which the sodium cations have been expelled or at least largely expelled from the polypyrrole, can be achieved at a voltage of 0 volts, i.e. no applied current. The reduced state, in which the sodium cations have moved into the polypyrrole, can be achieved, for example, at a voltage of about 1 volts, or perhaps about 1.2 volts. It should be noted that intermediate voltages, say in the range of 0.4 to 0.6 volts, can cause an intermediate level of volume increase as a result of cations migrating into the polymer. Depending on the voltage applied, the polypyrrole may achieve a volume increase of at least about 30 percent.

Depending on how the electroactive polymer 40 is employed, in some cases moving from the oxidized state to the reduced state, via application of an appropriate potential difference across the electroactive polymer, simply causes a volume increase, and the electroactive polymer merely swells or grows. In some cases, the electroactive polymer 40 may be coupled with an electrode, such as in a gold/polypyrrole bilayer, and moving between oxidized and reduced states may cause the bilayer to either bend or straighten.

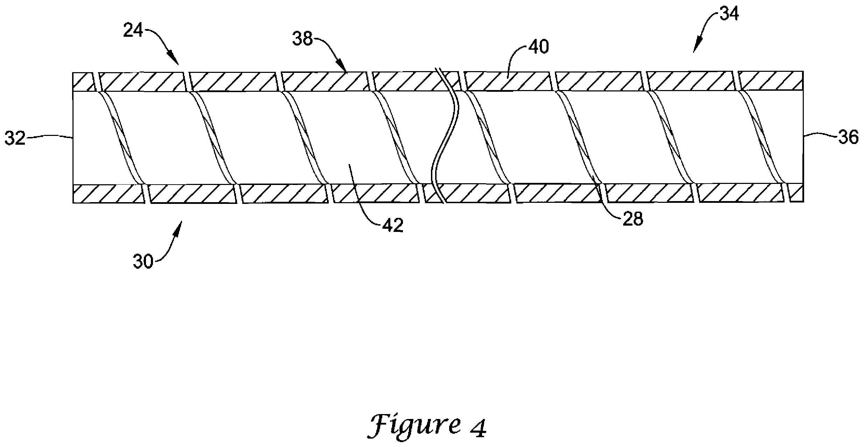

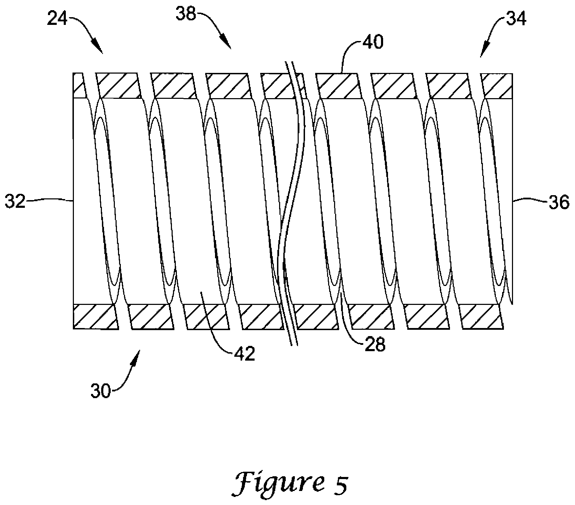

Returning now to the Figures, Figures 4 and 5 show the assembly 38 in an activated configuration in which a potential difference has been applied to the electroactive polymer 40. While no electrodes are shown in Figures 4 and 5, it will be appreciated that an electrical potential may be applied across the electroactive polymer 40 by providing a first conductive element on a first side of the electroactive polymer 40 and a second conductive element on a second, opposing, side of the electroactive polymer 40. In some cases, the first conductive element may be the hypotube body 26 or a wire disposed therein and the second conductive element may be exterior to the electroactive polymer 40 that is disposed on the hypotube body 26.

In some instances, as illustrated, it can be seen that activating the electroactive polymer 40 causes the electroactive polymer 40 to swell as ions migrate into the electroactive polymer in response to the applied current. As seen in Figure 4, the hypotube body 26 has constricted, or become more tightly wound, as the electroactive polymer 40 grew in volume. By comparing to Figure 3, it can be seen that the spiral cut hypotube 24 has a smaller outer diameter and helical cut 28 has reduced in size as a result of activating the electroactive polymer 40.

In some cases, it may be desirable to instead expand or unwind the hypotube body 26. As seen in Figure 5, the electroactive polymer 40 is adapted such that it causes the hypotube body 26 to unwind in response to the electroactive polymer 40 being activated. By comparing to Figure 3, it can be seen that the spiral cut hypotube 24 has a larger outer diameter and helical cut 28 has, in some cases, enlarged in size as a result of activating the electroactive polymer 40. In some cases, this configuration change may occur as a result of electroactive polymer 40 increasing in volume, as illustrated. In some instances, however, it is contemplated that the illustrated configuration may instead be obtained by reducing the volume of electroactive polymer 40, i.e. by providing a reverse current.

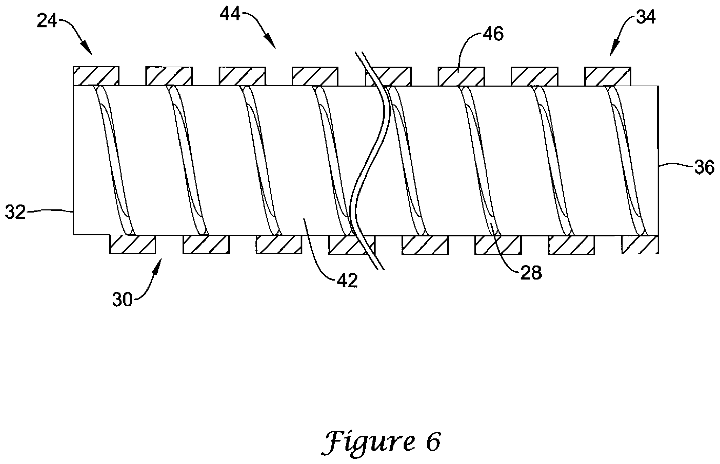

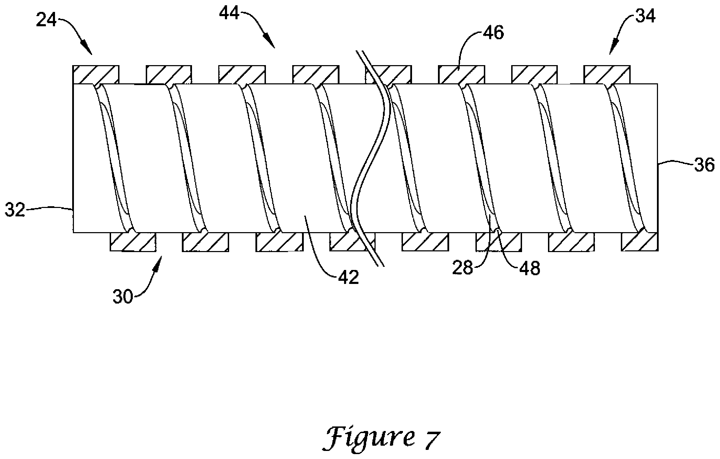

In Figures 3, 4 and 5, the electroactive polymer 40 was provided on an outer surface of the hypotube body 26 such that the helical cutting 28 was not covered or at least was not substantially covered. In some cases, it may be desirable for the electroactive polymer to span at least some sections of the helical cutting 28. Figures 6 and 7 provide illustrative but non-limiting examples of this.

Figure 6, not according to the invention, shows an assembly 44 in which an electroactive polymer 46 has been added to the spiral cut hypotube 24. In Figure 6, the electroactive polymer 46 has been disposed at least partially between at least some of the individual turnings 42 of the hypotube body 26 such that the electroactive polymer 44 spans at least some of the individual turnings of the helical cutting 28. While the electroactive polymer 46 is shown as covering substantially all of the individual turnings of the helical cutting 28, it will be recognized that the electroactive polymer 46 may instead be disposed only along a portion of the length of the hypotube body 26, or perhaps within two or more distinct segments or portions along the length of the hypotube body 26.

Figure 7 shows the assembly 44 in an activated configuration in which a potential difference has been applied to the electroactive polymer 46. While no electrodes are shown, it will be appreciated that an electrical potential may be applied across the electroactive polymer 46 by providing conductive electrodes on either side of the electroactive polymer 46. In some instances, as illustrated, it can be seen that activating the electroactive polymer 46 causes the electroactive polymer 46 to swell as ions migrate into the electroactive polymer in response to the applied current.

As shown, at least a portion 48 of the electroactive polymer 46 may expand at least partially into the helical cutting 28 when the electroactive polymer 46 expands in volume. In some cases, this may improve pushability and/or compressive strength by preventing the helical cutting 28 or portions thereof from narrowing in width. In some cases, this may improve pushability and/or compressive strength by limiting the ability of the spiral cut hypotube 24 to wind or unwind as a result of axial forces applied thereto.

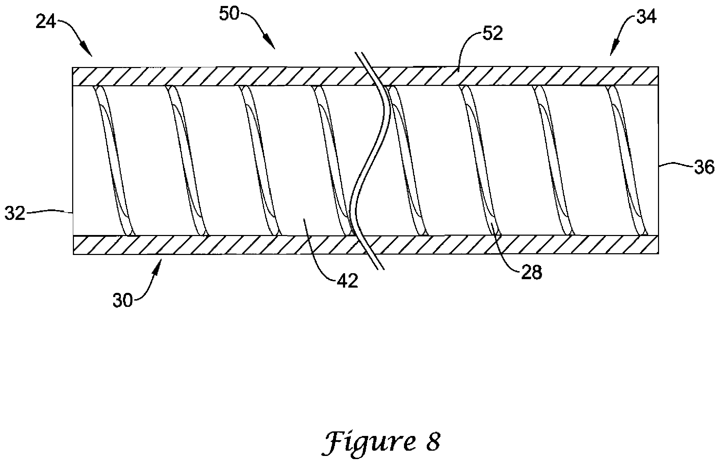

In Figures 6 and 7, the electroactive polymer 46 was disposed along the helical cutting 28 in one or more distinct ribbons or segments. In some instances, it may be useful for the electroactive polymer to cover a larger portion of an exterior surface of the spiral cut hypotube 24. Figure 8 provides an illustrative but non-limiting example of this.

Figure 8, not according to the invention, shows an assembly 50 in which an electroactive polymer 52 is disposed in a single continuous layer over hypotube body 26. In some cases, as illustrated, the electroactive polymer 52 may extend over at least a substantial number of the individual turnings 42 of the hypotube body 26 as well as over at least a substantial portion of the helical cutting 28. It will be appreciated that when the electroactive polymer 52 is activated as a result of applying an appropriate current, the electroactive polymer 52 may change in volume and as a result may improve the pushability and/or compressive strength of the spiral cut hypotube 24 by limiting the ability of the hypotube body 26 to wind or unwind as a result of axial forces applied thereto.

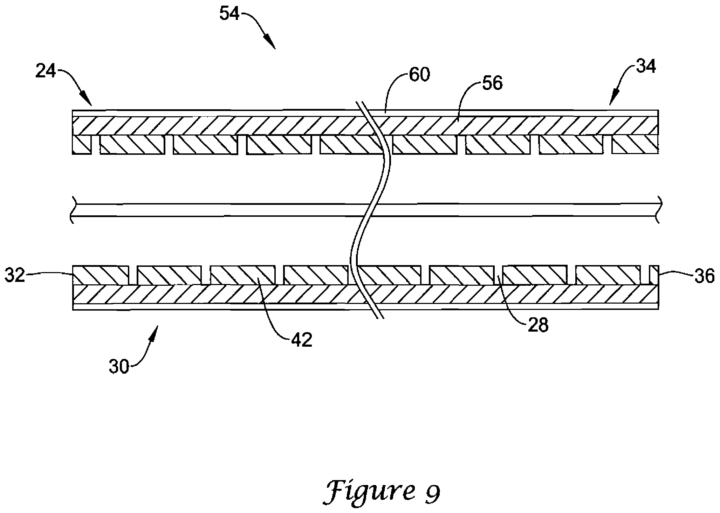

As referenced above, in order to apply a current to (or a potential difference across) the electroactive polymer 40, 46 and 52, two electrodes are needed. Figure 9 generally shows several ways to provide these electrodes. Figure 9 is a cross-sectional view of an assembly 54 in which an electroactive polymer 56 is disposed on the spiral cut hypotube 24. While shown as a single layer, it will be recognized that electroactive polymer 56 is intended to generally represent the electroactive polymer 40 shown in Figures 3, 4 and 5, the electroactive polymer 46 shown in Figures 6 and 7, and/or the electroactive polymer 52 shown in Figure 8.

In some cases, the spiral cut hypotube 24 may be made of a non-conductive or substantially non-conductive material such as a polymer or polymer blend. In such cases, it may be useful to provide a conductive lead 58 within an interior of the spiral cut hypotube 24. A conductive layer 60, generically shown exterior to the electroactive polymer 56, may function as a second electrode. In some cases, the conductive layer 60 may cover substantially all of the electroactive polymer 56. In some instances, it will be recognized that the conductive layer 60 may instead include two or more electrically isolated conductive regions that may be individually activated and as a result the properties of the assembly 54 (or any catheter or other medical device incorporating the assembly 54) may be more closely controlled.

In some instances, part or all of the devices described herein can include a lubricious coating. Lubricious coatings can improve steerability and improve lesion crossing capability. Examples of suitable lubricious polymers include hydrophilic polymers such as polyarylene oxides, polyvinylpyrolidones, polyvinylalcohols, hydroxy alkyl cellulosics, algins, saccharides, caprolactones, and the like, and mixtures and combinations thereof. Hydrophilic polymers can be blended among themselves or with formulated amounts of water insoluble compounds (including some polymers) to yield coatings with suitable lubricity, bonding, and solubility. In some embodiments, portions of the devices described herein can be coated with a hydrophilic polymer or a fluoropolymer such as polytetrafluoroethylene (PTFE), better known as TEFLON®.

The invention should not be considered limited to the particular examples described above, but rather should be understood to cover all aspects of the invention as set out in the attached claims. Various modifications, equivalent processes, as well as numerous structures to which the invention can be applicable will be readily apparent to those of skill in the art upon review of the instant specification.

## Claims

1. A catheter comprising:
an elongate shaft comprising a spiral cut hypotube with a helical cutting having a constant cutting pitch formed therein, the elongate shaft defining a lumen; and
an electroactive polymer disposed over at least a portion of the hypotube,
wherein the electroactive polymer is provided on an outer surface of the hypotube such that the helical cutting is not covered.

2. The catheter of claim 1, further comprising a conductive pattern disposed adjacent the hypotube.

3. The catheter of claim 2, wherein the hypotube defines the conductive pattern.

4. The catheter of claim 2, further comprising a conductive wire disposed within the lumen.

5. The catheter of claim 1, wherein the electroactive polymer is arranged to reversibly alter the cutting pitch of at least a portion of the hypotube.

6. The catheter of claim 1, wherein the hypotube has a pushability, and the electroactive polymer is arranged to reversibly alter the pushability of at least a portion of the hypotube.

7. The catheter of claim 1, wherein subjecting the electroactive polymer to a current causes available ions to move into the electroactive polymer, thereby causing the electroactive polymer to swell.

8. The catheter of claim 7, wherein the electroactive polymer comprises an electroactive polymer doped with an immobile anion.

9. The catheter of claim 8, wherein the electroactive polymer comprises polypyrrole and the immobile anion comprises sodium dodecyl benzene sulfonate.

10. The catheter of claim 7, wherein the available ions are provided in an electrolyte solution within the lumen.

11. The catheter of claim 7, wherein the available ions comprise a blood component available within a patient's blood.

## Patentansprüche

1. Katheter, der aufweist:
einen länglichen Schaft, der eine spiralförmig geschnittene Hypotube mit einem spiralförmigen Einschnitt aufweist, die eine darin ausgebildete konstante Einschnittteilung aufweist, wobei der längliche Schaft ein Lumen definiert; und
ein elektroaktives Polymer, das mindestens über einen Abschnitt der Hypotube angeordnet ist,
wobei das elektroaktive Polymer auf einer Außenfläche der Hypotube so vorgesehen ist, dass der spiralförmige Einschnitt nicht bedeckt ist.

2. Katheter nach Anspruch 1, der ferner ein leitfähiges Muster aufweist, das benachbart der Hypotube angeordnet ist.

3. Katheter nach Anspruch 2, wobei die Hypotube das leitfähige Muster definiert.

4. Katheter nach Anspruch 2, der ferner einen leitfähigen Draht aufweist, der innerhalb des Lumens angeordnet ist.

5. Katheter nach Anspruch 1, wobei das elektroaktive Polymer eingerichtet ist, die Einschnittteilung von mindestens einem Abschnitt der Hypotube reversibel zu ändern.

6. Katheter nach Anspruch 1, wobei die Hypotube eine Schubfähigkeit aufweist, und das elektroaktive Polymer eingerichtet ist, die Schubfähigkeit von mindestens einem Abschnitt der Hypotube reversibel zu ändern.

7. Katheter nach Anspruch 1, wobei das Anlegen eines Stroms an das elektroaktive Polymer bewirkt, dass sich verfügbare Ionen in das elektroaktive Polymer bewegen, wodurch bewirkt wird, dass das elektroaktive Polymer aufquillt.

8. Katheter nach Anspruch 7, wobei das elektroaktive Polymer ein mit einem unbeweglichen Anion dotiertes elektroaktives Polymer aufweist.

9. Katheter nach Anspruch 8, wobei das elektroaktive Polymer Polypyrrol aufweist und das unbewegliche Anion Natriumdodecylbenzensulfonat aufweist.

10. Katheter nach Anspruch 7, wobei die verfügbaren Ionen in einer Elektrolytlösung innerhalb des Lumen vorgesehen sind.

11. Katheter nach Anspruch 7, wobei die verfügbaren Ionen einen Blutbestandteil aufweisen, der im Blut eines Patienten verfügbar ist.

## Revendications

1. Cathéter, comprenant :
une tige allongée comportant un hypotube à découpe en spirale, présentant une découpe hélicoïdale avec un pas de coupe constant, ladite tige allongée définissant une lumière ;
et un polymère électroactif présent sur au moins une partie de l'hypotube,
où le polymère électroactif est prévu sur une surface extérieure de l'hypotube, de manière à ne pas couvrir la découpe hélicoïdale.

2. Cathéter selon la revendication 1, comprenant en outre un motif conducteur disposé de manière adjacente à l'hypotube.

3. Cathéter selon la revendication 2, où l'hypotube définit le motif conducteur.

4. Cathéter selon la revendication 2, comprenant en outre un fil conducteur à l'intérieur de la lumière.

5. Cathéter selon la revendication 1, où le polymère électroactif est disposé pour modifier de manière réversible le pas de coupe d'au moins une partie de l'hypotube.

6. Cathéter selon la revendication 1, où l'hypotube peut être poussé, et où le polymère électroactif est disposé pour modifier de manière réversible l'aptitude à la poussée d'au moins une partie de l'hypotube.

7. Cathéter selon la revendication 1, où l'application d'un courant sur le polymère électroactif provoque la pénétration d'ions disponibles dans le polymère électroactif, entraînant ainsi le gonflement du polymère électroactif.

8. Cathéter selon la revendication 7, où le polymère électroactif comprend un polymère électroactif dopé par un anion immobile.

9. Cathéter selon la revendication 8, où le polymère électroactif comprend du polypyrrole et l'anion immobile comprend du dodécylbenzènesulfonate de sodium.

10. Cathéter selon la revendication 7, où les ions disponibles sont prévus dans une solution d'électrolyte dans la lumière.

11. Cathéter selon la revendication 7, où les ions disponibles comprennent un composant sanguin disponible dans le sang d'un patient.
